(19) Europäisches Patentamt
European Patent Office
Office européen des brevets

(11) EP 0 937 719 B1

(12) **FASCICULE DE BREVET EUROPEEN**

(45) Date de publication et mention de la délivrance du brevet:
**10.04.2002 Bulletin 2002/15**

(51) Int Cl.[7]: **C07D 315/00**, C11B 9/00

(21) Numéro de dépôt: **99400127.9**

(22) Date de dépôt: **20.01.1999**

(54) **Procédé de fabrication de la cis-isoambrettolide et son application**

Verfahren zur Herstellung von CIS-Isoambrettolide und Ihre Anwendung

Process for the preparation of CIS-isoambrettoldide and her application

(84) Etats contractants désignés:
**AT BE CH CY DE DK ES FI FR GB GR IE IT LI LU MC NL PT SE**

(30) Priorité: **20.02.1998 FR 9802101**

(43) Date de publication de la demande:
**25.08.1999 Bulletin 1999/34**

(73) Titulaire: **Synarome**
**28000 Chartres (FR)**

(72) Inventeur: **Delphis, Claude**
**91940 les Ulis (FR)**

(74) Mandataire: **Keib, Gérard et al**
**NOVAMARK TECHNOLOGIES**
**Anciennement Brevets Rodhain & Porte**
**122, Rue Edouard Vaillant**
**92593 Levallois Perret Cedex (FR)**

(56) Documents cités:
**US-A- 4 014 902**

- **CHEMICAL ABSTRACTS, vol. 98, no. 13, 1983 Columbus, Ohio, US; abstract no. 107054w, MAJEE,RABINDRA ET AL.: "SYNTHESIS OF TRANS DELTA9-ISOAMBRETTOLIDE." page 578; colonne 2; XP002082048 & CHEM.IND.(LONDON), vol. 1, 1983, page 43 LONDON**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

EP 0 937 719 B1

## Description

La présente invention concerne un procédé de fabrication de la cis-isoambrettolide avec un très haut degré de pureté.

On sait que les substances à odeur musquée sont d'un usage courant dans l'industrie de la parfumerie au sens large où elles présentent un intérêt de premier plan, tant dans le domaine des parfums que dans celui des produits lessiviels tels que savons et détergents, du fait de leur effet de rémanence.

Ces molécules sont représentées par 4 familles : les muscs aromatiques non nitrés, les muscs aromatiques nitrés, les cétones macrocycliques et les lactones macrocycliques. Les lactones sont des aromatiques importants en raison de leur abondance dans les produits naturels.

Parmi les lactones macrocycliques, d'origine naturelle, provenant du règne végétal, on peut citer l'exaltolide, de formule (1) et l'ambrettolide, de formule (2).

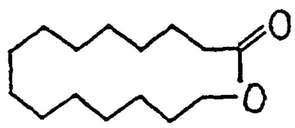
(1)

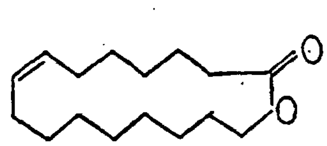
(2)

L'ambrettolide est obtenue à partir de l'huile essentielle et des résinoides d'ambrette dont la note ronde, chaude, musquée est très appréciée en parfumerie. Cette plante est l'Hibiscus abelmoschus 4, cultivée en Amérique du Sud, en Indonésie, aux Antilles.

On connaît déjà une lactone macrocyclique insaturée : la trans-isoambrettolide de formule (3), obtenue industriellement, qui est un isomère géométrique et de position de l'ambrettolide naturelle. Un procédé d'obtention de cette dernière est décrit dans le brevet US N° 4 014 902.

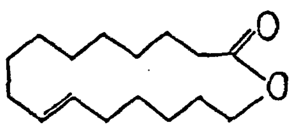
(3)

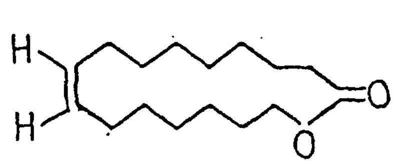
(4)

La présente invention concerne par contre un procédé de fabrication d'un autre isomère géométrique et de position de l'ambrettolide naturelle : la cis-isoambrettolide de formule (4), qui présente l'intérêt d'être olfactivement bien plus puissante que la trans isoambrettolide, ce qui permet de l'utiliser dans l'industrie de la parfumerie à des concentrations bien moins élevées, d'où une économie importante qui la fait d'autant plus apprécier.

En outre, l'intérêt du procédé, objet de la présente invention, réside dans le fait qu'il permet d'obtenir la cis-isoambrettolide avec un degré de pureté supérieur à 99,5 %, intérêt qui se conjugue avec le précédent.

L'acide cis 16-hydroxy 9-hexadécènoïque est un composé chimique essentiel en tant que produit de départ pour la synthèse de la lactone macrocyclique qu'est la cis-isoambrettolide, dont il est le précurseur direct.

La présente invention concerne donc un procédé de fabrication de l'acide cis 16-hydroxy 9-hexadécènoïque en vue de l'obtention ultérieure de la cis-isoambrettolide par les méthodes habituelles de macrolactonisation.

La présente invention concerne plus particulièrement un procédé de fabrication de la cis-isoambrettolide, mettant en oeuvre les étapes de réactions chimiques suivantes :

a) trans-époxydation de l'acide trans 16-hydroxy 9-hexadécènoïque, de formule (5), estérifié ou non, pour obtenir l'époxyde de formule (6) ;

b) ouverture du pont époxydique, pour obtenir l'acide érythro aleuritique, ou un ester de cet acide, de formule (7) ;

c) action d'un orthoester de formule HC $(OR'')_3$ sur l'acide érythro aleuritique, ou son ester, pour obtenir le dioxolanne, de formule (9) ;

d) pyrolyse du dioxolanne, pour obtenir l'acide cis 16-hydroxy 9-hexadécènoïque, ou un ester de cet acide, de formule (10), avec une pureté supérieure à 99 % ;

e) macrolactonisation de l'acide précédent, ou son ester, pour aboutir à la cis-isoambrettolide.

La présente invention a également pour objet, à titre de variante, un procédé de fabrication de la cis-isoambrettolide, mettant en oeuvre les étapes de réactions chimiques suivantes :

a) trans-époxydation de l'acide trans 16-hydroxy 9-hexadécènoïque, de formule (5), estérifié ou non, pour obtenir l'époxyde de formule (6) ;
b) ouverture du pont époxydique, pour obtenir un mélange de β-hydroxy éthers, de formule (8) ;
c) action d'un orthoester de formule $HC(OR'')_3$ sur le mélange de β-hydroxy éthers, pour obtenir le dioxolanne, de formule (9) ;
d) pyrolyse du dioxolanne, pour obtenir l'acide cis 16-hydroxy 9-hexadécènoïque, ou un ester de cet acide, de formule (10), avec une pureté supérieure à 99 % ;
e) macrolactonisation de l'acide précédent, ou son ester, pour aboutir à la cis-isoambrettolide.

Dans les formules précédentes :

$R^1$ représente un atome d'hydrogène ou le radical acétyle,
$R^2$ représente un atome d'hydrogène ou le radical méthyle,
R représente un atome d'hydrogène ou le radical méthyle,
R' représente le radical méthyle ou le radical éthyle,
R" représente le radical méthyle ou le radical éthyle.

Les exemples suivants, donnés à titre indicatif, d'une forme de réalisation, illustreront davantage le procédé de fabrication de la cis-isoambrettolide selon l'invention.
On pourra à cette occasion se référer aux figures en annexe, sur lesquelles :

- la figure 1 représente les différentes étapes du processus de fabrication de la cis-isoambrettolide,
- la figure 2 représente le spectre RMN $^1$H de la cis-isoambrettolide,
- la figure 3 représente le spectre IR de la cis-isoambrettolide.

Comme schématisé à la figure 1, les étapes de la préparation de l'acide cis 16-hydroxy 9-hexadécènoique, précurseur direct de la cis-isoambrettolide sont les suivantes :

a) préparation de l'époxyde, de formule (6), à partir de l'acide trans 16-hydroxy 9-hexadécènoïque, estérifié ou non, de formule (5), que l'on traite par le peroxyde d'hydrogène en milieu acide carboxylique tel que l'acide formique ou l'acide acétique, pour aboutir par trans-époxydation à l'époxyde de formule (6) ;
b) ouverture du pont époxydique, par hydrolyse in situ, en milieu acide ou basique, de l'époxyde, pour aboutir à l'acide érythro aleuritique ou à un ester de cet acide, de formule (7) ;
b') à titre de variante, ouverture du pont époxydique, par alcoolyse, de l'époxyde, qui conduit à un mélange de β-hydroxy éthers, de formule (8) ;
c) obtention du dioxolanne, de formule (9), par action d'un ortho ester de formule $HC(OR'')_3$, sur l'acide érythro aleuritique, ou son ester ;
c') à titre de variante, obtention du dioxolanne, par action d'un orthoester de formule $HC(OR'')_3$ sur le mélange de β-hydroxy éthers ;
d) obtention de l'acide cis 16-hydroxy 9-hexadécènoïque, ou son ester, de formule (10), par pyrolyse, en présence ou en l'absence de solvant, du dioxolanne.

On obtient enfin la cis-isoambrettolide par macrolactonisation de son acide précurseur.
Il est précisé que, pour l'ouverture du pont époxydique par alcoolyse, on fait appel à l'alcool méthylique ou l'alcool éthylique.

<u>EXEMPLE 1 - Obtention de la cis - isoambrettolide</u>

a) préparation de l'acide étythro aleuritique

Dans un réacteur de 2 litres, équipé d'un agitateur et d'un réfrigérateur externe, on charge 350 ml de peroxyde d'hydrogène à 30% et 280 ml d'acide formique. La température du mélange est abaissée à 0°C.

On rajoute alors 500 g d'acide trans 16-hydroxy 9-hexadécènoïque, à un rythme tel que la température du mélange ne dépasse pas 5°C. On règle ensuite la température à 10°C. On maintient le mélange sous agitation jusqu'à transformation totale en chromatographie de l'alcène.

On procède alors à la dilution du mélange par 1 litre d'eau. On laisse ensuite revenir la température à l'ambiante et l'on maintient l'agitation pendant encore une journée supplémentaire.

On effectue ensuite une extraction acido-basique de l'acide érythro aleuritique. On procède enfin à sa purification par cristallisation.

On notera que si l'on aboutit à un ester de l'acide érythro aleuritique, on procède à sa purification par distillation.

b) préparation de l'acide 16-hydroxy hexadécènoïque.

On traite à reflux par un orthoester, qui peut être le triméthylorthoformiate ou le triéthylorthoformiate, l'acide ou l'ester obtenu précédemment, jusqu'à transformation totale en chromatographie.

On procède ensuite à la pyrolyse in situ, du dioxolanne ainsi obtenu, en présence ou non d'un solvant, qui peut être le toluène ou le xylène, par élévation de la température jusqu'à 160°C.

On purifie par distillation l'ω-hydroxy-acide ou l'ω-hydroxyester ainsi obtenu (acide cis 16-hydroxy 9-hexadécènoïque ou un ester dudit acide).

c) obtention de la cis-isoambrettolide

On traite l'acide, ou son ester, précédemment obtenu dans les conditions habituelles de macrolactonisation, qui conduisent à la cis-isoambrettolide.

L'ensemble des étapes du processus de fabrication est représenté sur la figure 1.

La structure de la cis-isoambrettolide a été confirmée par la spectroscopie par résonance magnétique nucléaire du proton et par la spectroscopie infra-rouge, ainsi qu'on peut le voir sur les figures 2 et 3 respectivement, dont les résultats sont rassemblés ci-après :

- Analyse RMN$^1$H de la cis-isoambrettolide - solvant : $CDCl_3$

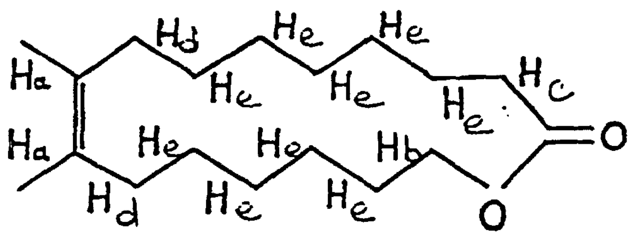

| H | δ(ppm) | intégrale | |
|---|---|---|---|
| Ha | 5,34 | 2 | couplage Ha-Ha : 8,06Hz |
| Hb | 4,12 | 2 | |
| Hc | 2,34 | 2 | |
| Hd | 2,03 | 4 | |
| He | 1,34 à 1,6 | 18 | |

- Analyse du spectre d'absorption infra-rouge

Les bandes d'absorption significatives se situent autour de :
2929 $cm^{-1}$ ; 2883 $cm^{-1}$ ; 1737 $cm^{-1}$ et 1460 $cm^{-1}$

La note ronde, musquée, de grande expansion de la cis-isoambrettolide en fait une matière première d'un très grand intérêt en parfumerie.

Elle appporte à toute composition parfumante le développement de la note, de sa diffusion, de sa rondeur et de sa tenue sans dénaturer l'ensemble de la note. Cet effet est perceptible dès des dosages faibles, de l'ordre de 0,3%, et très marquant pour des dosages de 1,5% à 5%.

On trouvera ci-après quelques exemples de compositions pour des parfums ou des articles parfumés tels que des eaux de toilette, des crèmes, des lotions, des savons, des détergents et des aérosols utilisant la cis-isoambrettolide.

<u>EXEMPLE 1</u> :

Ainsi, on l'utilise dans une note féminine florale à caractère jasmin, violette, ylang suivant la formule ci-après : (parties en poids)

| | |
|---|---|
| Acétate de benzyle | 6.00 |
| Acétate de styrallyle | 0.50 |
| Alcool cinnamique | 1.20 |
| Alcoole phényle éthylique | 16.00 |
| Aldéhyde C11 undécylénique 10% | 1.30 |
| Aldéhyde hexyl cinnamique | 3.50 |
| Bergamote défurocoumarinisée | 2.50 |
| Citronellol | 0.80 |
| Eugénol | 0.50 |
| Hédione | 0.70 |
| Hydroxycitronellal | 4.00 |
| Ionone 100% | 1.00 |
| Irisarome | 7.00 |
| Iso bornyl cyclohexanol | 2.50 |
| Cis-jasmone 10% | 0.50 |
| Linalol | 6.00 |
| Lyral | 1.00 |
| Méthyl cédryl cétone | 2.00 |
| Méthyl iso eugénol | 2.00 |
| Salicylate d'amyle | 2.00 |
| Salicylate de benzyle | 7.50 |
| Terpinéol | 3.50 |
| Vétivérol | 2.00 |
| Ylang 2$^{e}$ | 3.00 |
| Cis-isoambrettolide | 2.00 |
| | 79.00 |

La cis-isoambrettolide apporte à la note de l'expansion, une note plus ronde, plus sophistiquée et une tenue plus parfumée, musquée et poudrée.

EXEMPLE 2 :

On l'utilise aussi dans une note fraîche, hespéridée, boisée, ambrée, pour une eau de toilette unisexe ou masculine suivant la formule ci-après : (parties en poids)

| | |
|---|---|
| Acétal bois 12 | 7.40 |
| Acétate de cédrényle | 1.20 |
| Acétate d'isobornyle | 2.50 |
| Acétate de linalyle | 3.00 |
| Acétate de styrallyle | 0.50 |
| Adoxal 10% | 0.25 |
| Aldéhyde mandarine 10% | 0.25 |
| Allyl amyl glycolate | 0.60 |
| Ambrox DL 10% | 2.50 |
| Calone 161 10% | 0.30 |
| Carvone gauche 10% | 1.50 |
| Citron essence | 8.40 |
| Cyclogalbanate | 0.40 |
| Damascona alpha 10% | 4.00 |
| Diéthyl acétal du citral | 1.20 |
| Diméthyl cyclohexène carbaldéhyde 10% | 1.80 |
| Ethyl linalol | 8.00 |

(suite)

| | |
|---|---|
| Ethyl triméthyl cyclopentényl 10% | 1.20 |
| Evernyl | 0.30 |
| Hédione | 10.00 |
| Hélional | 1.70 |
| Hydroxycitronellal | 0.70 |
| Jessemal 10% | 2.00 |
| Lavadin essence | 3.00 |
| Mandarine essence | 1.00 |
| Menthe citrata | 0.10 |
| Méthylionone gamma | 0.50 |
| Orange essence | 5.00 |
| Cis-isoambrettolide | 6.00 |
| | 78.00 |

La cis-isoambrettolide apporte un caractère musqué, en développant l'ensemble de la note et tout particulièrement son aspect hespéridé, et favorise sa tenue.

Ces mêmes résultats de développement de la note, de sa tenue, avec un effet plus sophistiqué, sont constatés dans les compositions parfumantes destinées à des lignes de cosmétique, à des produits de toilette, à des shampooings et des produits pour la chevelure.

EXEMPLE 3 :

L'utilisation de la cis-isombrettolide est également très intéressante dans les compositions parfumantes destinées aux savons, détergents, nettoyants ménagers et assouplisseurs textiles.

Ainsi, dans la formule ci-après, à note fraîche tenace, à aspect fruité : (parties en poids)

| | |
|---|---|
| Acétate para tertio butyle cyclo hexyle | 6.00 |
| Acétate diméthyl benzyl carbinol | 0.70 |
| Acétate cis-3 hexényle | 0.05 |
| Acétate de styrallyle | 2.50 |
| Acétate de verdyle | 4.00 |
| Alcool phényl éthylique | 1.00 |
| Aldéhyde hexyl cinnamique | 10.00 |
| Algix 20% | 6.00 |
| Ambroxan 10% | 1.00 |
| Méthyl méthoxy phényl propanol | 0.60 |
| Cérinthol | 1.00 |
| Cétone alpha | 2.00 |
| Dihydroionone delta | 4.00 |
| Dihydro myrcénol | 7.30 |
| Eugénol | 0.20 |
| Floropal | 1.80 |
| Géraniol | 1.00 |
| Géranyl nitrile | 0.50 |
| Hédione | 10.00 |
| Cis 3 hexénol | 0.15 |
| Menthol | 0.30 |
| Méthyl cédryl cétone | 10.00 |
| Méthylionone gamma | 4.60 |
| Nonadiénal 2-6 DEA 1% | 1.00 |
| Orange essence | 4.50 |

(suite)

| | |
|---|---|
| Salicylate cis 3 hexényle | 1.00 |
| Undécalactone | 0.80 |
| Cis-isoambrettolide | 15.00 |
| | 97.00 |

La cis-isoambrettolide développe la note fraîche de cette composition tout au long de l'évaporation du produit et favorise très nettement sa tenue, sa substantivité et développe une note musquée.

Bien que la présente description de l'invention ait été faite en référence à des formes particulières de réalisations, il est bien clair que l'on peut apporter diverses modifications opératoires mineures, qui sont à la portée immédiate de l'homme de l'art, sans sortir pour autant du cadre de l'invention telle que décrite et revendiquée.

## Revendications

**1.** Procédé de fabrication de la cis-isoambrettolide, à un très haut degré de pureté, **caractérisé en ce que** l'on effectue à titre intermédiaire la préparation de l'acide cis-16-hydroxy 9-hexadécènoïque, ou un ester de cet acide, précurseur direct de la cis-isoambrettolide, selon les étapes chimiques suivantes :

a) trans-époxydation de l'acide trans 16-hydroxy 9-hexadénènoïque, estérifié ou non, de formule (5), pour obtenir l'époxyde de formule (6), dans laquelle $R^1$ est un atome d'hydrogène ou le radical acétyle et $R^2$ est un atome d'hydrogène ou le radical méthyle ;
b) ouverture du pont époxydique, pour obtenir l'acide érythro aleuritique, ou un ester de cet acide, de formule (7) ;
c) action d'un orthoester de formule $HC(OR'')_3$ sur l'acide érythro aleuritique, ou son ester, de formule (7) pour obtenir le dioxolanne, de formule (9) ; avec R" qui représente le radical méthyle ou le radical éthyle ;
d) pyrolyse du dioxolanne de formule (9), pour obtenir l'acide cis 16-hydroxy 9-hexadécènoïque, ou un ester de cet acide, de formule (10). (Voir schéma 1).

SCHÉMA 1

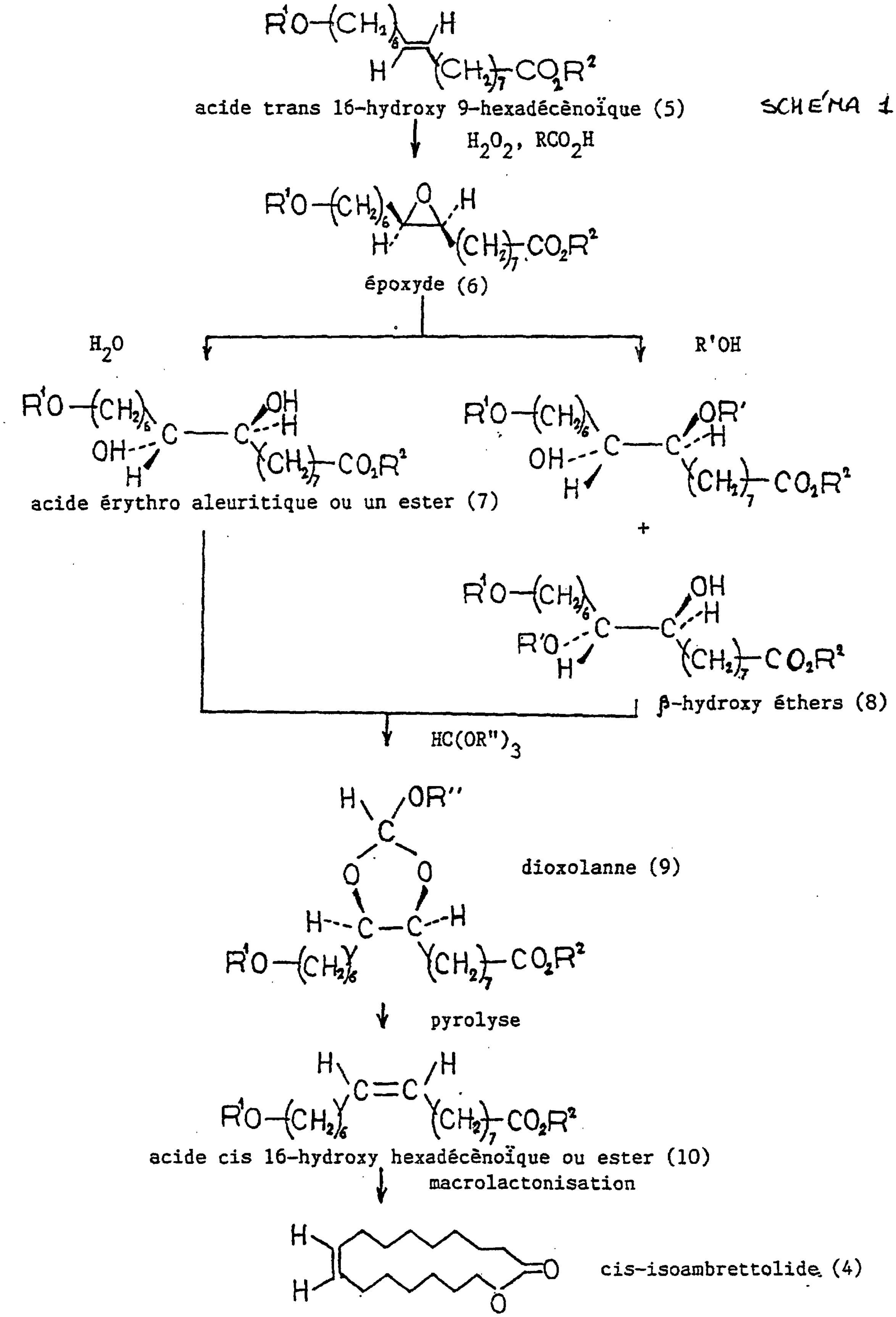


acide trans 16-hydroxy 9-hexadécènoïque (5)

époxyde (6)

acide érythro aleuritique ou un ester (7)

+

β-hydroxy éthers (8)

dioxolanne (9)

acide cis 16-hydroxy hexadécènoïque ou ester (10)

cis-isoambrettolide (4)

**2.** Procédé de fabrication de la cis-isoambrettolide, à un très haut degré de pureté, **caractérisé en ce que** l'on effectue à titre intermédiaire la préparation de l'acide cis-16-hydroxy 9-hexadécènoïque, ou un ester de cet acide, précurseur direct de la cis-isoambrettolide, selon les étapes chimiques suivantes :

a) trans-époxydation de l'acide trans 16-hydroxy 9-hexadénènoïque, estérifié ou non, de formule (5), pour obtenir l'époxyde de formule (6), dans laquelle $R^1$ est un atome d'hydrogène ou le radical acétyle et $R^2$ est un atome d'hydrogène ou le radical méthyle ;
b) ouverture du pont époxydique, pour obtenir un mélange de β-hydroxy éthers, de formules (8), dans lesquelles $R^1$ est un atome d'hydrogène ou le radical acétyle, $R^2$ est un atome d'hydrogène ou le radical méthyle et R' est le radical méthyle ou le radical éthyle ;
c) action d'un ortho ester de formule $HC(OR'')_3$ sur le mélange de β-hydroxy éthers de formules (8), pour obtenir le dioxolanne, de formule (9) ; avec R" qui représente le radical méthyle ou le radical éthyle ;
d) pyrolyse du dioxolanne de formule (9), pour obtenir l'acide cis 16-hydroxy 9-hexadécènoïque, ou un ester de cet acide, de formule (10).

**3.** Procédé de fabrication de la cis-isoambrettolide selon la revendication 1 ou 2, **caractérisé en ce qu'**il comprend en outre l'étape suivante :

e) macrolactonisation de l'acide cis 16-hydroxy 9-hexadécènoïque, ou de son ester, de formule (10), pour aboutir à la cis-isoambrettolide (4). (Voir schéma 1).

**4.** Procédé de fabrication de la cis-isoambrettolide selon l'une quelconque des revendications 1 à 3, **caractérisé en ce que** la trans-époxydation de l'acide trans 16-hydroxy 9-hexadécènoïque, estérifié ou non, de formule (5), de l'étape a) s'effectue par traitement par le peroxyde d'hydrogène en présence d'un acide carboxylique.

**5.** Procédé de fabrication de la cis-isoambrettolide selon la revendication 4, **caractérisé en ce que**, dans la trans-époxydation de l'étape a), l'acide carboxylique est l'acide formique ou l'acide acétique.

**6.** Procédé de fabrication de la cis-isoambrettolide selon la revendication 1 ou l'une quelconque des revendications dépendantes de la revendication 1, **caractérisé en ce que** l'ouverture du pont époxydique de l'étape b) s'effectue par hydrolyse in situ en milieu acide.

**7.** Procédé de fabrication de la cis-isoambrettolide selon la revendication 1 ou l'une quelconque des revendications dépendantes de la revendication 1, **caractérisé en ce que** l'ouverture du pont époxyde de l'étape b) s'effectue par hydrolyse in situ en milieu basique.

**8.** Procédé de fabrication de la cis-isoambrettolide selon la revendication 2 ou l'une quelconque des revendications dépendantes de la revendication 2, **caractérisé en ce que** l'ouverture du pont époxydique de l'étape b) s'effectue par alcoolyse, pour obtenir le mélange de β-hydroxy éthers de formules (8).

**9.** Procédé de fabrication de la cis-isoambrettolide selon la revendication 8, **caractérisé en ce que**, dans l'ouverture du pont époxydique de l'étape b) par alcoolyse, on fait appel à l'alcool méthylique ou l'alcool éthylique.

**10.** Procédé de fabrication de la cis-isoambrettolide selon l'une quelconque des revendications 1 à 9, **caractérisé en ce que** l'orthoester de l'étape c) peut être le triméthylorthoformiate ou le triéthylorthoformiate.

**11.** Procédé de fabrication de la cis-isoambrettolide selon l'une quelconque des revendications 1 à 10, **caractérisé en ce que** la pyrolyse du dioxolanne de formule (9) de l'étape d) s'effectue en présence d'un solvant.

**12.** Procédé de fabrication de la cis-isoambrettolide selon la revendication 11, **caractérisé en ce que** le solvant peut être le toluène ou le xylène.

**13.** Procédé de fabrication de la cis-isoambrettolide selon l'une quelconque des revendications 1 à 10, **caractérisé en ce que** la pyrolyse du dioxolanne de formule (9) de l'étape d) s'effectue en l'absence de solvant.

**14.** Procédé de fabrication de la cis-isoambrettolide selon l'une quelconque des revendications 1 à 13, **caractérisé en ce que** l'acide cis 16-hydroxy 9-hexadécènoïque, estérifié ou non, de formule (10), est obtenu dans l'étape d) avec une pureté supérieure à 99%.

**15.** Procédé de fabrication de la cis-isoambrettolide selon l'une quelconque des revendications 1 à 14, **caractérisé en ce que** la cis-isoambrettolide de formule (4) est obtenue avec une pureté d'isomère géométrique supérieure à 99,5%.

**16.** Utilisation de la cis-isoambrettolide de formule (4) en tant que matière première de parfumage dans les compositions parfumantes destinées à des parfums et eaux de toilette, à des lignes cosmétiques, à des produits de toilette, à des shampooings, à des produits pour la chevelure, à des savons, détergents, assouplissants textiles, nettoyants ménagers et à tout produit parfumé.

**17.** Procédé de préparation de l'acide cis-16-hydroxy 9-hyxadénènoïque, ou un ester de cet acide, **caractérisé en ce qu'**il comprend les étapes chimiques suivantes :

a) trans-époxydation de l'acide trans 16-hydroxy 9-hexadénènoïque, estérifié ou non, de formule (5), pour obtenir l'époxyde de formule (6), dans laquelle $R^1$ est un atome d'hydrogène ou le radical acétyle et $R^2$ est un atome d'hydrogène ou le radical méthyle ;
b) ouverture du pont époxydique, pour obtenir l'acide érythro aleuritique, ou un ester de cet acide, de formule (7) ;
c) action d'un orthoester de formule $HC(OR'')_3$ sur l'acide érythro aleuritique, ou son ester, de formule (7), pour obtenir le dioxolanne, de formule (9) ; avec R'' qui représente le radical méthyle ou le radical éthyle ;
d) pyrolyse du dioxolanne de formule (9), pour obtenir l'acide cis 16-hydroxy 9-hexadécènoïque, ou un ester de cet acide, de formule (10).

**18.** Procédé de préparation de l'acide cis 16-hydroxy 9-hexadénènoïque, ou un ester de cet acide, selon la revendication 17 **caractérisé en ce que** ledit procédé comprenant les étapes chimiques suivantes :

a) trans-époxydation de l'acide trans 16-hydroxy 9-hexadénènoïque, estérifié ou non, de formule (5), pour obtenir l'époxyde de formule (6), dans laquelle $R^1$ est un atome d'hydrogène ou le radical acétyle et $R^2$ est un atome d'hydrogène ou le radical méthyle ;
b) ouverture du pont époxydique, pour obtenir un mélange de β-hydroxy éthers de formules (8), dans lesquelles $R^1$ est un atome d'hydrogène ou le radical acétyle, $R^2$ est un atome d'hydrogène ou le radical méthyle et R' est le radical méthyle ou le radical éthyle ;
c) action d'un ortho ester de formule $HC(OR'')_3$ sur le mélange de β-hydroxy éthers de formules (8), pour obtenir le dioxolanne, de formule (9) ; avec R'' qui représente le radical méthyle ou le radical éthyle ;
d) pyrolyse du dioxolanne de formule (9), pour obtenir l'acide cis 16-hydroxy 9-hexadécènoïque, ou un ester de cet acide, de formule (10).

**19.** Procédé de préparation de l'acide cis 16-hydroxy 9-hexadénènoïque, ou un ester de cet acide, selon la revendication 17 ou 18, **caractérisé en ce que** l'acide cis 16-hydroxy 9-hexadécènoïque, estérifié ou non, de formule (10), est obtenu dans l'étape d) avec une pureté supérieure à 99%.

## Claims

**1.** A process for the production of cis-isoambrettolide of a very high degree of purity, **characterised in that** cis 16-hydroxy 9-hexadecenoic acid or an ester of that acid is prepared intermediately, the same being a direct precursor of cis-isoambrettolide, in accordance with the following chemical steps:

a) trans-epoxidation of trans 16-hydroxy 9-hexadenenoic acid, esterified or not, of formula (5) to obtain the epoxide of formula (6), wherein $R^1$ is a hydrogen atom or the acetyl radical and $R^2$ is a hydrogen atom or the methyl radical;

b) opening of the epoxide bridge to obtain erythro-aleuritic acid, or an ester of that acid, of formula (7);

c) action of an orthoester of formula $HC(OR'')_3$ on the erythro-aleuritic acid, or its ester, of formula (7) to obtain dioxolan of formula (9); with R'' which represents the methyl radical or the ethyl radical;

d) pyrolysis of the formula (9) dioxolan to obtain cis 16-hydroxy 9-hexadecenoic acid, or an ester of that acid, of formula (10). (See diagram 1).

**DIAGRAM 1**

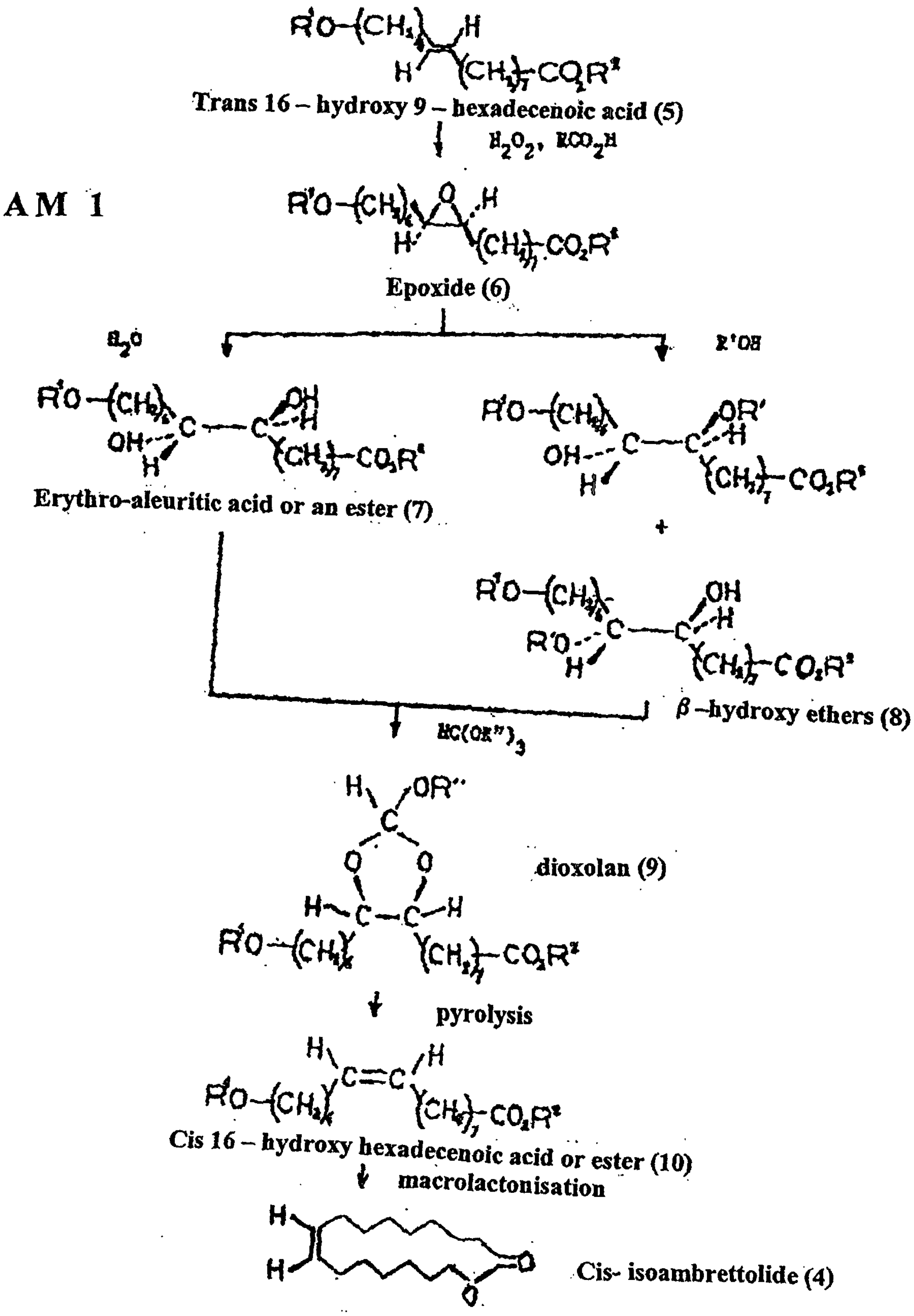


Trans 16 – hydroxy 9 – hexadecenoic acid (5)

Epoxide (6)

Erythro-aleuritic acid or an ester (7)

+

β-hydroxy ethers (8)

dioxolan (9)

Cis 16 – hydroxy hexadecenoic acid or ester (10)

Cis- isoambrettolide (4)

**2.** A process for the production of cis-isoambrettolide of a very high degree of purity, **characterised in that** cis 16-hydroxy 9-hexadecenoic acid or an ester of that acid is prepared intermediately, the same being a direct precursor of cis-isoambrettolide, in accordance with the following chemical steps:

a) trans-epoxidation of trans 16-hydroxy 9-hexadenenoic acid, esterified or not, of formula (5) to obtain the epoxide of formula (6), wherein $R^1$ is a hydrogen atom or the acetyl radical and $R^2$ is a hydrogen atom or the methyl radical;

b) opening of the epoxide bridge to obtain a mixture of β-hydroxy ethers, of formulae (8) wherein $R^1$ is a hydrogen atom or the acetyl radical, $R^2$ is a hydrogen atom or the methyl radical and R' is the methyl radical or the ethyl radical;

c) action of an ortho-ester of formula $HC(OR'')_3$ on the mixture of β-hydroxy ethers of formulae (8) to obtain dioxolan of formula (9); with R" which represents the methyl radical or the ethyl radical;

d) pyrolysis of the formula (9) dioxolan to obtain cis 16-hydroxy 9-hexadecenoic acid, or an ester of that acid, of formula (10).

**3.** A process for the production of cis-isoambrettolide according to claim 1 or 2, **characterised in that** it also comprises the following step:

e) macrolactonisation of the cis 16-hydroxy 9-hexadecenoic acid, or its ester, of formula (10), to obtain cis-isoambrettolide (4). (See diagram 1).

**4.** A process for the production of cis-isoambrettolide according to any one of claims 1 to 3, **characterised in that** the trans-epoxidation of the trans 16-hydroxy 9-hexadecenoic acid, esterified or not, of formula (5), of step a) is effected by treatment by hydrogen peroxide in the presence of a carboxylic acid.

**5.** A process for the production of cis-isoambrettolide according to claim 4, **characterised in that** in the trans-epoxidation of step a), the carboxylic acid is formic acid or acetic acid.

**6.** A process for the production of cis-isoambrettolide according to claim 1 or any one of the claims dependent on claim 1, **characterised in that** opening of the epoxide bridge of step b) is effected by hydrolysis in situ in an acid medium.

**7.** A process for the production of cis-isoambrettolide according to claim 1 or any one of the claims dependent on claim 1, **characterised in that** the opening of the epoxide bridge of step b) is effected by hydrolysis in situ in a basic medium.

**8.** A process for the production of cis-isoambrettolide according to claim 2 or any one of the claims dependent on claim 2, **characterised in that** the opening of the epoxide bridge of step b) is effected by alcoholysis, to obtain the mixture of β-hydroxyethers of formulae (8).

**9.** A process for the production of cis-isoambrettolide according to claim 8, **characterised in that** in the opening of the epoxide bridge of step b) by alcoholysis, use is made of methyl alcohol or ethyl alcohol.

**10.** A process for the production of cis-isoambrettolide according to any one of claims 1 to 9, **characterised in that** the ortho-ester of step c) may be trimethylorthoformiate or triethylorthoformiate.

**11.** A process for the production of cis-isoambrettolide according to any one of claims 1 to 10, **characterised in that** the pyrolysis of the formula (9) dioxolan of step d) is effected in the presence of a solvent.

**12.** A process for the production of cis-isoambrettolide according to claim 11, **characterised in that** the solvent may be toluene or xylene.

**13.** A process for the production of cis-isoambrettolide according to any one of claims 1 to 10, **characterised in that** the pyrolysis of the dioxolan of formula (9) of step d) is effected in the absence of a solvent.

**14.** A process for the production of cis-isoambrettolide according to any one of claims 1 to 13, **characterised in that** the cis 16-hydroxy 9-hexadecenoic acid, esterified or not, of formula (10), is obtained in step d) with a purity greater than 99%.

**15.** A process for the production of cis-isoambrettolide according to any one of claims 1 to 14, **characterised in that** the cis-isoambrettolide of formula (4) is obtained with a geometric isomer purity greater than 99.5%.

**16.** Use of the cis-isoambrettolide of formula (4) as a perfumery raw material in perfumery compositions intended for

toilet waters and perfumes, for cosmetic lines, for toilet products, for shampoos, for hair care products, for soaps, detergents, textile softeners, household cleaners, and any perfumed product.

**17.** A process for the preparation of cis 16-hydroxy 9-hexadecenoic acid, or an ester of that acid, **characterised in that** it comprises the following chemical steps:

a) trans-epoxidation of trans 16-hydroxy 9-hexadecenoic acid, esterified or not, of formula (5) to obtain the epoxide of formula (6), wherein $R^1$ is a hydrogen atom or the acetyl radical and $R^2$ is a hydrogen atom or the methyl radical;

b) opening of the epoxide bridge to obtain erythro-aleuritic acid, or an ester of that acid, of formula (7);

c) action of an orthoester of formula $HC(OR'')_3$ on the erythro-aleuritic acid, or its ester, of formula (7) to obtain dioxolan of formula (9); with R" which represents the methyl radical or the ethyl radical;

d) pyrolysis of the formula (9) dioxolan to obtain cis 16-hydroxy 9-hexadecenoic acid, or an ester of that acid, of formula (10).

**18.** A process for the preparation of cis 16-hydroxy 9-hexadecenoic acid, or an ester of that acid, according to claim 17, **characterised in that** it comprises the following chemical steps:

a) trans-epoxidation of trans 16-hydroxy 9-hexadecenoic acid, esterified or not, of formula (5) to obtain the epoxide of formula (6), wherein $R^1$ is a hydrogen atom or the acetyl radical and $R^2$ is a hydrogen atom or the methyl radical;

b) opening of the epoxide bridge to obtain a mixture of β-hydroxy ethers, of formulae (8) wherein $R^1$ is a hydrogen atom or the acetyl radical, $R^2$ is a hydrogen atom or the methyl radical and R' is the methyl radical or the ethyl radical;

c) action of an ortho-ester of formula $HC(OR'')_3$ on the mixture of β-hydroxy ethers of formulae (8) to obtain dioxolan of formula (9); with R" which represents the methyl radical or the ethyl radical;

d) pyrolysis of the formula (9) dioxolan to obtain the cis 16-hydroxy 9-hexadecenoic acid, or an ester of that acid, of formula (10).

**19.** A process for the preparation of cis 16-hydroxy 9-hexadecenoic acid, or an ester of that acid, according to claim 17 or 18, **characterised in that** the cis 16-hydroxy 9-hexadecenoic acid, esterified or not, of formula (10) is obtained in step d) with a purity greater than 99%.

**Patentansprüche**

**1.** Verfahren zur Herstellung von cis-Isoambrettolid mit sehr hohem Reinheitsgrad, **dadurch gekennzeichnet, daß** als Zwischenstufe die Herstellung von cis-16-Hydroxy-9-hexadecensäure oder einem Ester dieser Säure als direktem Vorläufer von cis-Isoambrettolid gemäß den folgenden chemischen Schritten erfolgt:

a) trans-Epoxidierung von trans-16-Hydroxy-9-hexadecensäure, verestert oder nicht, der Formel (5), um das Epoxid der Formel (6) zu erhalten, bei der $R^1$ ein Wasserstoffatom oder Acetylrest ist und $R^2$ ein Wasserstoffatom oder Methylrest ist;

b) Öffnung der Epoxidbrücke, um erythro-Aleuritinsäure oder einen Ester dieser Säure der Formel (7) zu erhalten;

c) Reaktion eines Orthoesters der Formel $HC(R'')_3$ mit der erythro-Aleuritinsäure oder deren Ester gemäß Formel (7), um das Dioxolan der Formel (9) zu erhalten, wobei R" den Methylrest oder den Ethylrest darstellt;

d) Pyrolyse des Dioxolans der Formel (9), um die cis-16-Hydroxy-9-hexadecensäure oder einen Ester dieser Säure der Formel (10) zu erhalten (siehe Schema 1).

Schema 1

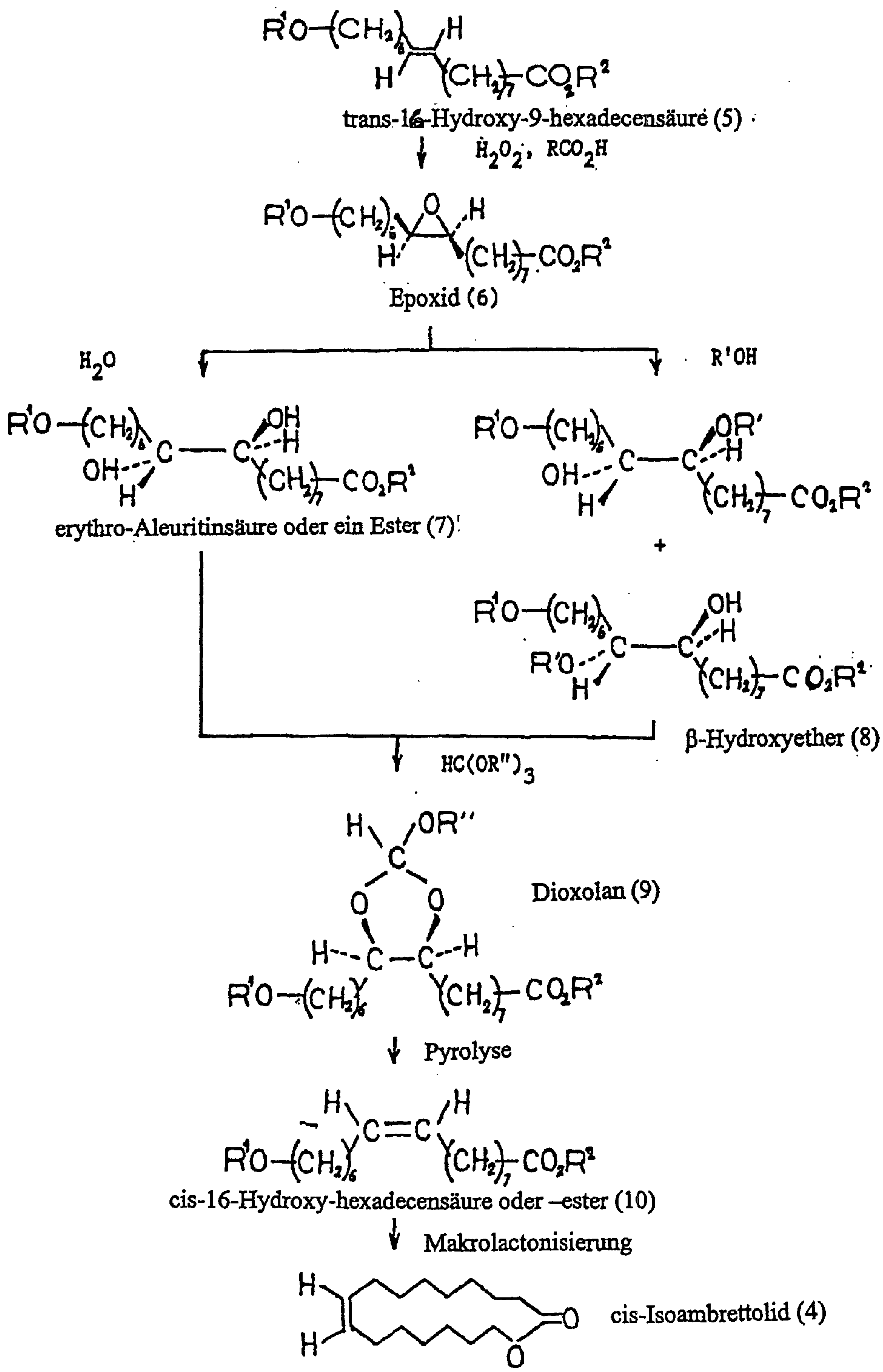


trans-16-Hydroxy-9-hexadecensäure (5)

Epoxid (6)

erythro-Aleuritinsäure oder ein Ester (7)

+

β-Hydroxyether (8)

Dioxolan (9)

cis-16-Hydroxy-hexadecensäure oder –ester (10)

cis-Isoambrettolid (4)

**2.** Verfahren zur Herstellung von cis-Isoambrettolid mit sehr hohem Reinheitsgrad, **dadurch gekennzeichnet, daß** als Zwischenstufe die Herstellung von cis-16-Hydroxy-9-hexadecensäure oder einem Ester dieser Säure als direktem Vorläufer von cis-Isoambrettolid gemäß den folgenden chemischen Schritte erfolgt:

a) trans-Epoxidierung der trans-16-Hydroxy-9-hexadecensäure, verestert oder nicht, nach Formel (5), um das Epoxid der Formel (6) zu erhalten, bei der $R^1$ ein Wasserstoffatom oder Acetylrest ist und $R^2$ ein Wasserstoffatom oder Methylrest ist;

b) Öffnung der Epoxidbrücke, um eine Mischung der β-Hydroxyether der Formeln (8) zu erhalten, bei denen $R^1$ ein Wasserstoffatom oder Acetylrest ist, $R^2$ ein Wasserstoffatom oder Methylrest ist und R' ein Methylrest oder Ethylrest ist;

c) Reaktion eines Orthoesters der Formel $HC(OR'')_3$ mit der Mischung der β-Hydroxyether der Formeln (8), um das Dioxolan der Formel (9) zu erhalten, bei der R" den Methylrest oder Ethylrest darstellt;

d) Pyrolyse des Dioxolans der Formel (9), um die cis-16-Hydroxy-9-hexadecensäure oder einen Ester dieser Säure der Formel (10) zu erhalten.

**3.** Verfahren zur Herstellung des cis-Isoambrettolids nach Anspruch 1 oder 2, **dadurch gekennzeichnet, daß** es unter anderem den folgenden Schritt umfaßt: Macrolactonisierung der cis-16-Hydroxy-9-hexadecensäure oder ihres Esters der Formel (10), um zum cis-Isoambrettolid (4) zu gelangen (siehe Schema 1).

**4.** Verfahren zur Herstellung des cis-Isoambrettolids nach einem der Ansprüche 1 bis 3, **dadurch gekennzeichnet, daß** die trans-Epoxidierung der trans-16-Hydroxy-9-hexadecensäure, verestert oder nicht, der Formel (5), in Schritt a) durch Reaktion mit Wasserstoffperoxid in Gegenwart einer Carbonsäure abläuft.

**5.** Verfahren zur Herstellung von cis-Isoambrettolid nach Anspruch 4, **dadurch gekennzeichnet, daß** während der trans-Epoxidierung in Schritt a) die Carbonsäure Ameisensäure oder Essigsäure ist.

**6.** Verfahren zur Herstellung von cis-Isoambrettolid nach Anspruch 1 oder einem der von Anspruch 1 abhängigen Ansprüche, **dadurch gekennzeichnet, daß** die Öffnung der Epoxidbrücke in Schritt b) durch in situ Hydrolyse im sauren Milieu abläuft.

**7.** Verfahren zur Herstellung von cis-Isoambrettolid nach Anspruch 1 oder einem der von Anspruch 1 abhängigen Ansprüche, **dadurch gekennzeichnet, daß** die Öffnung der Epoxidbrücke in Schritt b) durch in situ Hydrolyse im basischen Milieu abläuft.

**8.** Verfahren zur Herstellung von cis-Isoambrettolid nach Anspruch 2 oder einem der von Anspruch 2 abhängigen Ansprüche, **dadurch gekennzeichnet, daß** die Öffnung der Epoxidbrücke in Schritt b) durch Umesterung abläuft, um die Mischung aus β-Hydroxyethern der Formeln (8) zu erhalten.

**9.** Verfahren zur Herstellung von cis-Isoambrettolid nach Anspruch 8, **dadurch gekennzeichnet, daß** während der Öffnung der Epoxidbrücke in Schritt b) durch Umesterung Methylalkohol oder Ethylalkohol verwendet wird.

**10.** Verfahren zur Herstellung von cis-Isoambrettolid nach einem der Ansprüche 1 bis 9, **dadurch gekennzeichnet, daß** der Orthoester in Schritt c) Trimethylorthoformiat oder Triethylorthoformiat sein kann.

**11.** Verfahren zur Herstellung von cis-Isoambrettolid nach einem der Ansprüche 1 bis 10, **dadurch gekennzeichnet, daß** die Pyrolyse des Dioxolans der Formel (9) in Schritt d) in Gegenwart eines Lösungsmittels abläuft.

**12.** Verfahren zur Herstellung von cis-Isoambrettolid nach Anspruch 11, **dadurch gekennzeichnet, daß** das Lösungsmittel Toluol oder Xylol ist.

**13.** Verfahren zur Herstellung von cis-Isoambrettolid nach einem der Ansprüche 1 bis 10, **dadurch gekennzeichnet, daß** die Pyrolyse des Dioxolans der Formel (9) in Schritt d) in Abwesenheit von Lösungsmittel abläuft.

**14.** Verfahren zur Herstellung von cis-Isoambrettolid nach einem der Ansprüche 1 bis 13, **dadurch gekennzeichnet, daß** die cis-16-Hydroxy-9-hexadecensäure, verestert oder nicht, nach Formel (10) in Schritt d) mit einer Reinheit

von mehr als 99 % erhalten wird.

**15.** Verfahren zur Herstellung von cis-Isoambrettolid nach einem der Ansprüche 1 bis 14, **dadurch gekennzeichnet, daß** das cis-Isoambrettolid der Formel (4) mit einer Isomerenreinheit von mehr als 99,5 % erhalten wird.

**16.** Verwendung von cis-Isoambrettolid der Formel 4 als Parfümrohstoff in duftenden Zusammensetzungen zur Verwendung als Parfum und Eaux de Toilette, für Kosmetikserien, für Mittel zur Schönheitspflege, für Haarwaschmittel, für Haarpflegeprodukte, für Seifen, Detergentien, Textilweichmacher, Haushaltsreiniger und alle parfümierten Erzeugnisse.

**17.** Verfahren zur Herstellung von cis-16-Hydroxy-9-hexadecensäure oder einem Ester dieser Säure, **dadurch gekennzeichnet, daß** es die folgenden chemischen Schritte umfaßt:

a) trans-Epoxidierung der trans-16-Hydroxy-9-hexadecensäure, verestert oder nicht, der Formel (5), um das Epoxid der Formel (6) zu erhalten, bei der $R^1$ ein Wasserstoffatom oder Acetylrest ist oder $R^2$ ein Wasserstoffatom oder Methylrest ist;

b) Öffnung der Epoxidbrücke, um die erythro-Aleuritinsäure oder einen Ester dieser Säure der Formel (7) zu erhalten;

c) Reaktion eines Orthoesters der Formel $HC(OR'')_3$ mit der erythro-Aleuritinsäure oder ihrem Ester der Formel (7), um das Dioxolan der Formel (9) zu erhalten, bei der R" den Methylrest oder den Ethylrest darstellt;

d) Pyrolyse des Dioxolans der Formel (9), um die cis-16-Hydroxy-9-hexadecensäure oder einen Ester dieser Säure der Formel (10) zu erhalten.

**18.** Verfahren zur Herstellung der cis-16-Hydroxy-9-hexadecensäure oder einem Ester dieser Säure nach Anspruch 17, **dadurch gekennzeichnet, daß** das Verfahren die folgenden chemischen Schritte umfaßt:

a) trans-Epoxidierung der trans-16-Hydroxy-9-hexadecensäure, verestert oder nicht, der Formel (5), um das Epoxid der Formel (6) zu erhalten, bei der $R^1$ ein Wasserstoffatom oder Ethylrest ist und $R^2$ ein Wasserstoffatom oder Methylrest ist;

b) Öffnung der Epoxidbrücke, um eine Mischung der β-Hydroxyether der Formeln (8) zu erhalten, bei denen $R^1$ ein Wasserstoffatom oder Acetylrest ist, $R^2$ ein Wasserstoffatom oder Methylrest ist und R' ein Methylrest oder Ethylrest ist;

c) Reaktion eines Orthoesters der Formel $HC(OR'')_3$ mit der Mischung der β-Hydroxyether der Formeln (8), um das Dioxolan der Formel (9) zu erhalten, bei der R" den Methylrest oder den Ethylrest darstellt;

d) Pyrolyse des Dioxolans der Formel (9), um die cis-16-Hydroxy-9-hexadecensäure oder einen Ester dieser Säure der Formel (10) zu erhalten.

**19.** Verfahren zur Herstellung der cis-16-Hydroxy-9-hexadecensäure oder einem Ester dieser Säure nach Anspruch 17 oder 18, **dadurch gekennzeichnet, daß** die cis-16-Hydroxy-9-hexadecensäure, verestert oder nicht, der Formel (10) in Schritt d) mit einer Reinheit von mehr als 99 % erhalten wird.

**Fig.1**

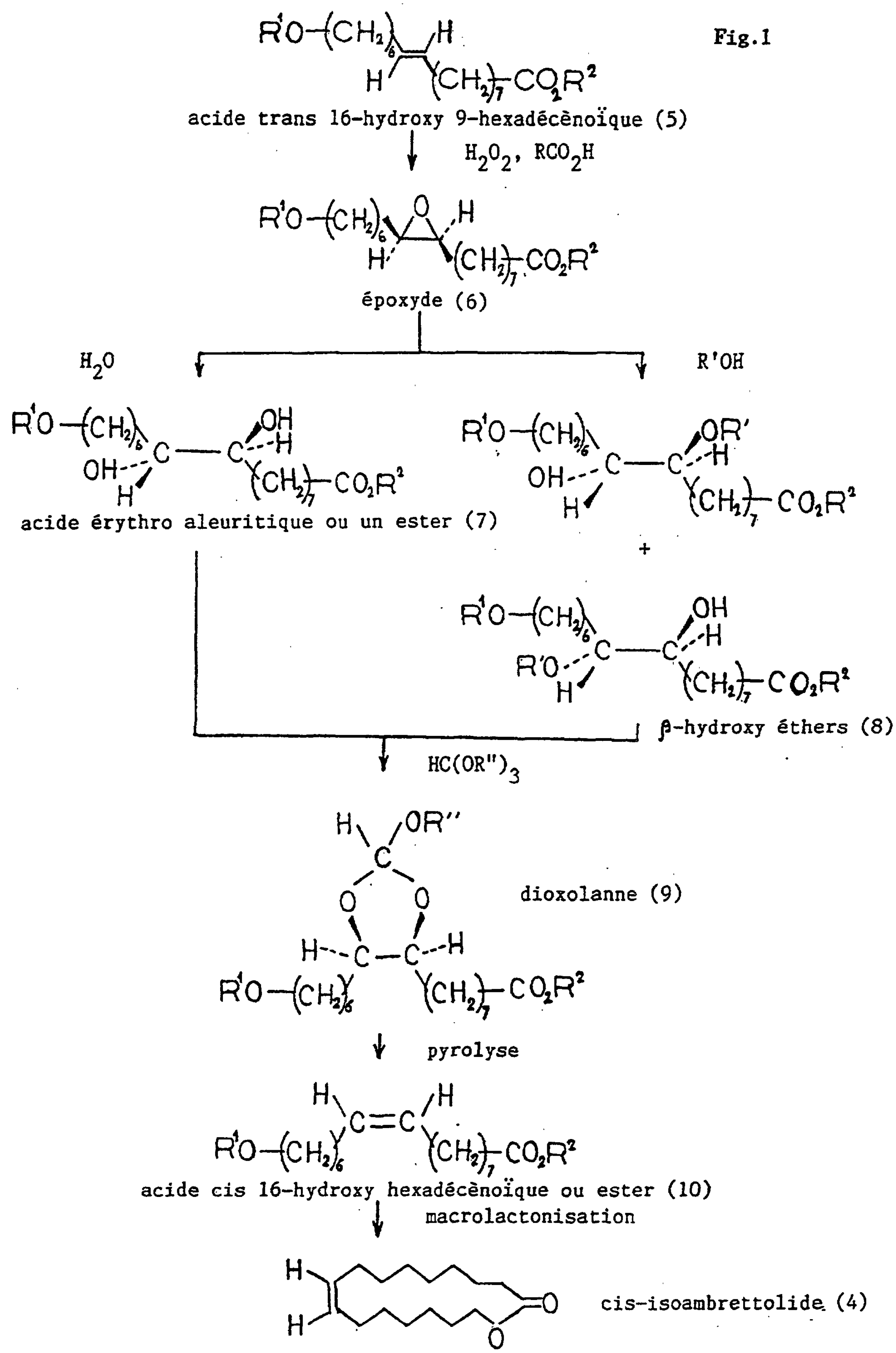


acide trans 16-hydroxy 9-hexadécènoïque (5)

époxyde (6)

acide érythro aleuritique ou un ester (7)

+

β-hydroxy éthers (8)

dioxolanne (9)

acide cis 16-hydroxy hexadécènoïque ou ester (10)

cis-isoambrettolide (4)

RMN $^1H$ CIS-ISOAMBRETTOLIDE

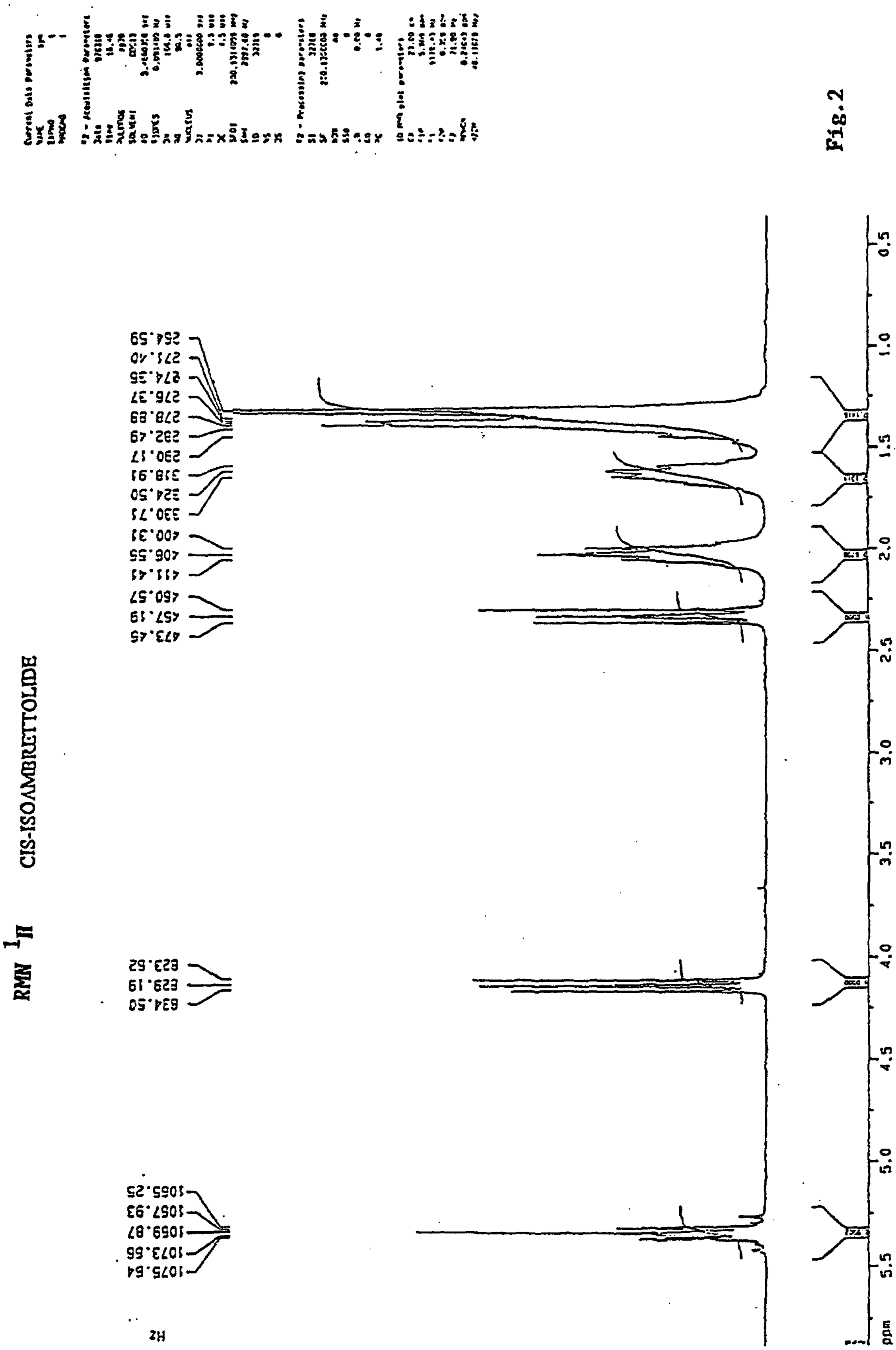


Fig.2

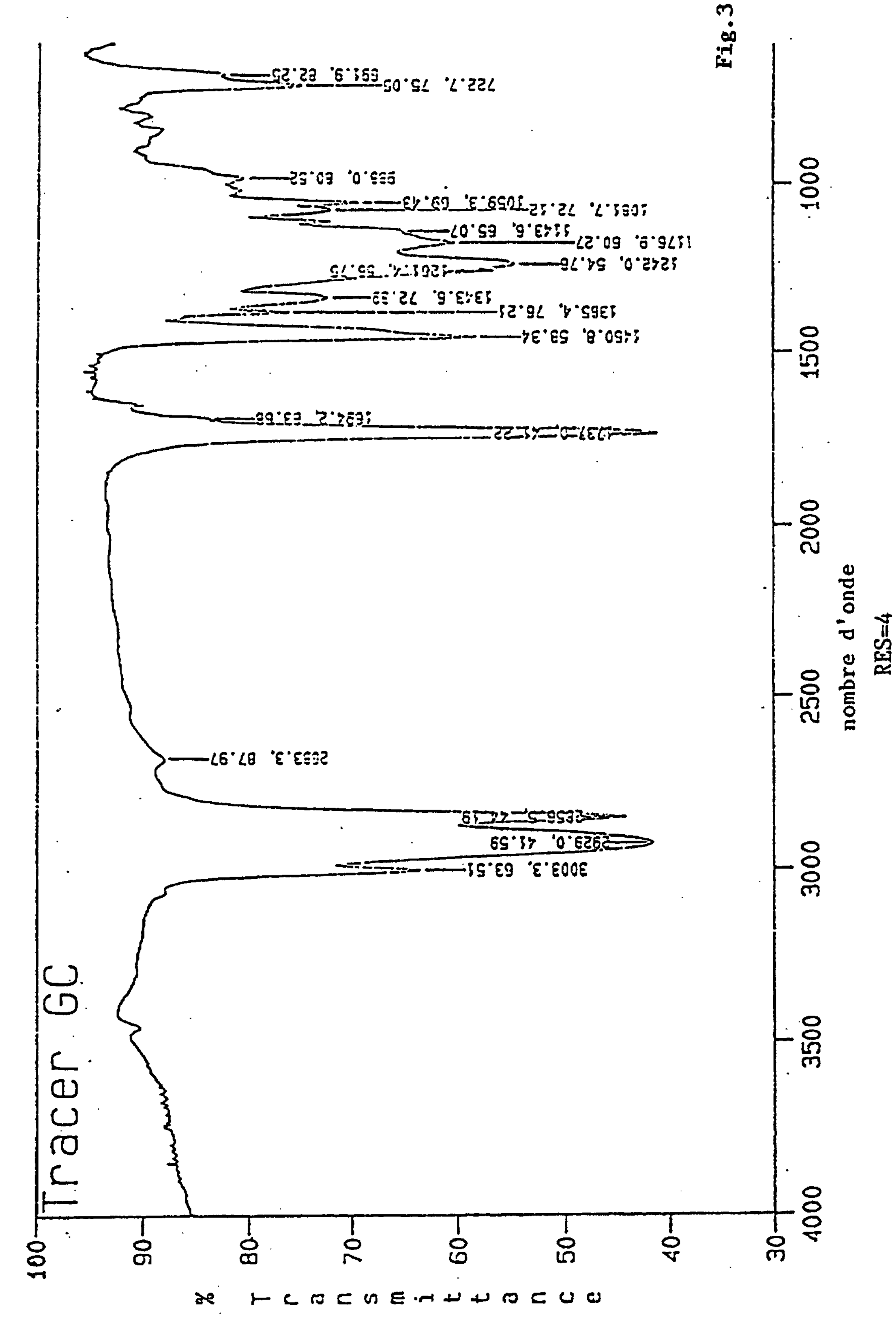
Tracer GC
% Transmittance
100
90
80
70
60
50
40
30
4000
3500
3000
2500
2000
1500
1000
nombre d'onde
RES=4
3003.3, 63.51
2929.0, 41.59
2856.5, 44.19
2653.3, 87.97
1737.0, 41.22
1692.2, 63.68
1450.8, 59.34
1365.4, 76.21
1343.6, 72.33
1261.4, 55.75
1242.0, 54.76
1176.9, 60.27
1143.6, 65.07
1081.7, 72.12
1059.3, 69.43
955.0, 60.52
722.7, 75.05
691.9, 82.25

Fig.3